# EUROPÄISCHE PATENTSCHRIFT

(11) **EP 0 757 554 B1**
(45) Veröffentlichungstag und Bekanntmachung des Hinweises auf die Patenterteilung: **04.08.1999**
(21) Anmeldenummer: 95913840.5
(22) Anmeldetag: 07.04.1995
(51) Int. Cl.: A61K 7/16

(54) **LUTSCHBONBON MIT ZAHNBELAGS-NEUTRALISIERENDER WIRKUNG**
LOLLIPOP WITH TOOTH PLAQUE NEUTRALISING EFFECT
SUCETTE A ACTION ANTITARTRE

(30) Priorität: 22.04.1994 CH 125194
(43) Veröffentlichungstag der Anmeldung: 12.02.1997
(73) Patentinhaber: DIBONA HOLDING AG, 6300 Zug (CH)
(72) Erfinder: KAUFMANN, Konrad, CH-8953 Dietikon (CH)
(74) Vertreter: Troesch Scheidegger Werner AG
(86) Internationale Anmeldenummer: CH9500078
(87) Internationale Veröffentlichungsnummer: WO9528910

(56) Entgegenhaltungen:
- EP-A- 0 525 388
- US-A- 4 409 202
- US-A- 5 250 569
- PATENT ABSTRACTS OF JAPAN vol. 11 no. 361 (C-459) & JP,A,62 132815 (TANAKA KIICHI) 16.Juni 1987,
- DATABASE WPI Week 8602 Derwent Publications Ltd., London, GB; AN 86-011915 & JP-A-60 237 947 (SAN EI CHEM IND KK) , 26.November 1985

## Beschreibung

Die vorliegende Erfindung betrifft ein Lutschbonbon mit Zahnbelags-neutralisierender Wirkung, die Verwendung eines Lutschbonbons zum Reinigen von Zähnen, sowie ein Verfahren und eine Vorrichtung zum Herstellen eines Lutschbonbons. Unter Lutschbonbons werden auch generell ovale Komprimate, Kaubonbons, Pastillen, Kapseln, Tabletten und ähnliche Darreichungsformen verstanden.

Der Zahnbelag ist nach der Einnahme von Nahrungs- und Genussmitteln infolge der dabei entstehenden Säuren besonders gefährdet. Insbesondere Zucker bewirken und fördern Zahnkaries und es gilt heute als wissenschaftlich gesichert, dass kariöse Läsionen das Ergebniss eines von aussen auf die Zahnhartsubstanzen einwirkenden Prozesses sind. Die Vergärung von oral zugeführten Kohlenhydraten zu oranischen Säuren in den bakteriellen Zahnbelägen, den Plaque, und die damit verbundene pH-Senkung dieser Beläge sind bei der Entstehung der Karies von zentraler Bedeutung.

Zur Neutralisierung von Zahnbelagsäuren werden alkalische Verbindungen, wie beispielsweise Natriumcarbonat, Bicarbonat, Amoniumphosphate, sowie Harnstoffe empfohlen, um organische Säuren, welche durch die Vergärung von Kohlenhydraten entstehen entgegen zu wirken. Der salzige und teilweise bittere Geschmack dieser Pufferungen hat jedoch dazu gefuhrt, dass deren Einsatz schwer realisierbar ist.

An sich die wirkungsvollste, mechanische Methode zum Hemmen von Karies besteht darin, die Zähne nach jeder Einnehme von Genuss- bzw. Nahrungsmitteln mit Zahnpasten bzw. Zahnbürsten zu reinigen. Allerdings ist es im allgemeinen nicht immer möglich, nach jeder Mahlzeit bzw. nach jedem Verzehr von Lebensmitteln die Zähne mit Zahnpaste und Bürste zu reinigen. Sei dies, dass man unterwegs ist, dass man auf Reisen ist, dass man am Arbeitsplatz ist, usw.

Aus der EP-525 388 wird die Herstellung und Zusammensetzung einer Tablette beschrieben mit dem Ziel der Erzeugung einer leichten Brausewirkung zur Geschmacksverbesserung von Tabletten mit pharmazeutischen Wirkstoffen, vor allem Kalzium und Magnesium. In der US-4 409 202 wird eine Tablette oder ein Bonbon beschrieben, deren Ziel es ist Mundgeruch zu überdecken oder zu neutralisieren. Hauptwirkstoff ist ein pflanzliches Oel. In der US-5 250 569 wird die Verwendung von Aminosäuren, unter anderem im Zusammenhang mit Zahnpasten vorgeschlagen, um den unangenehmen Geschmack von oral zugeführten Aluminiumverbindungen zu vermeiden. Speziell aluminiumhaltige Rhinitismedikationen sollen damit produzierbar sein. Ein weiteres Ziel dieses US-Patentes besteht im Erzeugen eines sogenannten Slow-release-Effektes für Aluminium. Ziel der JP-A-60237947 ist die galenische Herstellung einer bestimmten Erscheinungsform für ein Bonbon. Durch Einschluss von Luftblasen soll ein spezielles Aussehen im Endprodukt erreicht werden, welche Luftblasen offenbar einen Erfrischungseffekt im Bonbon erzielen sollen. In der JP-A-62132815 schliesslich wird die Herstellung von Tabletten zur Mundreinigung und zur Atemdesodorierung vorgeschlagen. Die aus dem Stand der Technik bekannten und vorgeschlagenen Massnahmen sind jedoch nicht geeignet Karies wirkungsvoll zu hemmen.

Auf diesem Hintergrund werden seit einiger Zeit sogenannte Plaque-Neutralisations-Kaugummis angeboten, welche einerseits den Speichelfluss infolge der Kaubewegung anregen und zum andern die oben erwähnten Wirksubstanzen beinhalten, welche eine Pufferung im Speichel bewirken, bzw. welche zur Neutralisation der bei der Gärung von Kohlenhydraten entstehenden Säuren führen. Diese Kaugummis haben den Vorteil, dass sie unmittelbar nach dem Essen angewendet werden können, wodurch zumindest zu einem grossen Teil, die die Zähne schädigenden Säuren neutralisiert werden können. Sei dies durch Stimulation des Speichelflusses, wodurch eine erhöhte Pufferkapazität erzeugt wird, durch Förderung der Neutralisation der Plaquesäure, durch verstärkte Speichelverteilung in schwer erreichbare Interdentalräume, durch verbesserte Reinigung der Mundhöhle von Nahrungsresten, durch Schaffung eines pH-Wertes, der die Remineralisation des Zahnschmelzes fördert oder schlussendlich durch Förderung der Remineralisation durch den stimulierten Speichelfluss mit erhöhtem Mineralgehalt.

Diese Kaugummis sind auch nützlich, beispielsweise für Patienten mit zeitlich begrenzter, oder bleibender Einschränkung der Mobilität, was in der Regel zu einer verminderten Qualität der Mundhygiene führt. Auch bei der Einnahme von Medikamenten, welche zu einer Hemmung des Speichelflusses führen, kann der erwähnte Kaugummi ein nützliches Instrument bilden, die Mundhygiene zu verbessern.

Das Problem von diesen Plaque-Neutralisations-Kaugummis liegt nun aber darin, dass das Kaugummikauen bei sehr vielen Leuten sozial nicht akzeptiert ist, oder aber aus anderen Gründen, wie beispielsweise beim Vorhandensein von Zahnprotesen, Kunststoffzähnen, etc., abgelehnt wird.

Es ist daher eine Aufgabe der vorliegenden Erfindung eine Alternative zum sogenannten Kaugummi zu schaffen, wodurch eine erhöhte Neutralisation der sauren Zahnbeläge ermöglicht wird, womit die Entstehung von Karies bei Zahnbelägen verhindert bzw. zumindest gehemmt werden kann.

Erfindungsgemäss wird die gestellte Aufgabe mittels einem Lutschbonbon gemäss dem Wortlaut nach Anspruch 1 gelöst.

Vorgeschlagen wird ein Lutschbonbon mit Plaque-neutralisierender Wirkung, welches mindestens einen weitgehendst säureneutralisierenden Wirkstoff enthält, wie beispielsweise bekannt von den erwähnten Kaugummis.

Der Vorteil der erfindungsgemäss vorgeschlagenen Lutschbonbons liegt einerseits darin, dass die oben im Zusammenhang mit Kaugummis dargestellte Problematik entfällt. Im weiteren werden ja bekanntlich bei der Verwendung von Kaugummis die darin enthaltenen Wirkstoffe innerhalb der ersten 1 - 2 Minuten durch das kräftige Kauen freigelegt, womit nach einer Dauer von ca. 2 Minuten nur noch wenig neutralisationswirksame Substanzen mehr durch den Kaugummi freigegebenen werden und natürlich die neutralisierende Wirkung reduziert wird. Bei einem Lutschbonbon hingegen ist die Zudosierung der neutralisierenden Wirkstoffe besser verteilt und kann bis zu einer Zeitdauer von 5 - 6 Minuten reichen. Die Anregung des Speichelflusses ist im übrigen nahezu identisch mit derjenigen bei der Verwendung von Kaugummis.

Als Wirkstoffe werden vorgeschlagen Amoniumsalze, Alkalimetallsalze, Erdalkalimetallsalze, wie insbesondere Phosphate, Carbonate, Hydrogencarbonate, Hydrogenphosphate, sowie Harnstoff oder Harnstoff-Peroxid.

Um den gelegentlich salzigen, seifigen oder bitteren Geschmack dieser säureneutralisierenden Wirkstoffe zu überdecken werden vorzugsweise dem erfindungsgemässen Lutschbonbon Zuckerersatzstoffe bzw. Zuckeraustauschstoffe und Süssstoffe beigefügt. In diesem Zusammenhang sind beispielsweise zu nennen: Isomalt, Sorbit, Acesulfam, Lycasin, zyklamathaltige Süssstoffe, Aspartam, usw.

Im weiteren hat es sich als vorteilhaft erwiesen, zusätzlich zum säureneutralisierenden Wirkstoff Xylit beizugeben, da ebenfalls Xylit eine gewisse Hemmwirkung auf die Zahnbelagsbildung, insbesondere zusammen mit dem erwähnten Wirkstoff ergibt.

Nebst den erwähnten Substanzen bzw. Wirkstoffen enthalten die erfindungsgemässen Lutschbonbons mindestens eine Trägersubstanz, sowie ein oder mehrere geschmacksbildende Aromastoffe, wie beispielsweise Pfefferminzaroma, Orangenaroma, etc.

Der Anteil an säureneutralisierenden Wirkstoffen sollte mindestens 0,1 Gew.% im erfindungsgemässen Lutschbonbon betragen, vorzugsweise zwischen 0,2 und 15 Gew.%, bezogen auf das Gesamtgewicht des Bonbons. Allerdings hat es sich in der Praxis gezeigt, dass der Anteil dieser säureneutralisierenden Wirkstoffe zwischen 0,5 und 3 Gew.%, bzw. vorzugsweise 1 bis 2,5 Gew.% liegen soll, ansonsten die "Neutralisierung" des bitteren, salzigen oder seifigen Geschmackes schwierig wird.

Gemäss einer bevorzugten Ausführungsvariante des erfindungsgemässen Lutschbonbons wird vorgeschlagen, als säureneutralisierenden Wirkstoff Di-Amoniumhydrogenphosphat und Natriumhydrogencarbonat bzw. eine Mischung davon zu verwenden.

Grundsätzlich ist der Gedanke neu, ein Lutschbonbon zum Neutralisieren von Zahnbelägen zu verwenden, bzw. ein Lutschbonbon mit einem neutralisierenden Wirkstoff zu versehen.

Zur Herstellung eines erfindungsgemässen Lutschbonbons wird vorgeschlagen, ein grundsätzlich bekanntes Verfahren zur Herstellung von Bonbons zu verwenden, wobei jedoch vorgeschlagen wird, dass bei der Zudosierung der säureneutralisierenden Wirkstoffe in die Bonbonherstellungsmasse einen Temperaturbereich zu wählen, der 135°C nicht übersteigt. Es hat sich in der Praxis gezeigt, dass in der Regel bei Temperaturen höher als die genannten 135 °C die erfindungswesentlichen Wirksubstanzen mindestens teilweise zersetzt bzw. abgebaut werden.

Aus diesem Grunde wird erfindungsgemäss eine Vorrichtung bzw. eine Anlage zur Herstellung eines erfindungsgemässen Lutschbonbons vorgeschlagen, wobei grundsätzlich von einer üblicherweise für die Herstellung von Lutschbonbons verwendeten Vorrichtung bzw. Anlage ausgegangen wird. Erfindungswesentlich ist nun, dass anschliessend an das Kochgefäss, wie beispielsweise eine Kochschlange, wo die verschiedenen Basismaterialien, wie Trägersubstanzen, Wasser, Zuckerersatzstoffe und dgl. bei ca. 160°C gekocht und gemischt werden, anschliessend diese Masse mittels eines Zusatzkühlers auf eine Temperatur von ca. 130 - 135°C abgekühlt wird. Dieser zusätzlich vorgeschlagene Kühler ist üblicherweise in derartigen Anlagen nicht vorgesehen. Anschliessend an diese zusätzliche Kühlung erfolgt das Zudosieren der säureneutralisierenden Wirkstoffe, wie oben vorgeschlagen.

Weitere bevorzugte Eigenschaften der Erfindung sind in den abhängigen Ansprüchen 2 bis 8 beansprucht.

Die Erfindung wird nun anschliessend anhand eines Beispieles näher erläutert, wobei anhand von Versuchen mittels der erfindungsgemässen Neutralisationsbonbons bei einer Reihe von Testpersonen am Zahnärztlichen Institut der Universität Zürich die neutralisierende Wirkung von Bonbons erhärtet worden ist. Die vom Zahnärztlichen Institut der Universität Zürich ermittelten Resultate sind anhand der beigefügten Figuren näher erläutert, bzw. erhärtet, wobei
- Fig. 1: die neutralisierende Wirkung eines Bonbons zeigt, auf durch Saccharosevergärung angesäuerten interdentalen Plaquebelägen, sowie
- Fig. 2: einen Vergleich der neutralisierenden Wirkung zwischen einem erfindungsgemässen Lutschbonbon bzw. Neutralisationsbonbon und einem bekannten Zahnreinigungskaugummi.

Die Anmelderin der vorliegenden Erfindung erteilte der Abteilung für Präventivzahnmedizin, Paradontologie und Kariologie des Zahnärztlichen Institutes der Universität Zürich den Auftrag, ein Muster des erfindungsgemäss beschriebenen Neutralisationsbonbons auf seine zahnschonenden Eigenschaften und auf seine Plaque-neutralisierende Wirkung verschiedenen Tests zu unterziehen.

Die Rezeptur des verwendeten Neutralisationsbonbons beinhaltete die nachfolgende Zusammensetzung:

| Rezeptur NEUTRALISATIONS BONBONS | | | |
|---|---|---|---|
| Rohstoffe | Einwaage in kg | Einwaage im Endprodukt mit ca.99% TS | Einwaage in 100 g/% Bonbon à 1.8g |
| Wasser | 183.8333 | 10.1238 | 1.0000 |

| Zuckeraustauschstoffe: | | | |
|---|---|---|---|
| Isomalt 95% | 1,038.1666 | 986.2583 | 97.4195 |
| Xylit CM 100% | 6.5000 | 6.5000 | 0.6420 |
| Di-Amoniumhydrogenphosphat | 4.0000 | 4.0000 | 0.3951 |
| Natriumhydrogencarbonat | 2.5000 | 2.5000 | 0.2469 |
| Pfefferminzaroma | 2.0000 | 2.0000 | 0.1975 |
| Acesulfam K (Kein Abbau) | 1.0000 | 1.0000 | 0.0987 |
| | 1,237.9999 | 1,012.3821 | 99.9997 |

Die Prüfungen erfolgten mit 8 Probanden bei bester Gesundheit. Sie hatten bereits bei früheren Tests teilgenommen und ihre physiologischen Mundhöhlenverhältnisse waren den Untersuchern genau bekannt. Durchschnittswerte der individuellen Plaque-pH-Kurven dieser Versuchspersonen nach 10% Saccharose-Spülungen, ermittelt während mehreren Jahren, sind zum Vergleich in der Computer-Datenbank des Zahnärztlichen Institutes gespeichert. Alle Probanden besassen eine Unterkiefer-Telemetrieprothese mit je einer, in einem Interproximalraum eingebauten Miniatur-pH-Glaselektrode.

Die Testprothesen wurden gereinigt eingesetzt und die Probanden angewiesen, während der Testdauer ihre normalen Essgewohnheiten beizubehalten, sich jedoch jeglicher Hundhygiene im Unterkiefer zu enthalten. Wasserspülungen zur Nahrungsrestentfernung und der Gebrauch einer Zahnbürste ohne Zahnpasta im Oberkiefer waren gestattet. Die Nichtentfernung der Prothesen erlaubte ein ungestörtes Plaquewachstum auf den Membran-Oberflächen der interdental eingebauten Elektroden. Dabei wurden die beiden nachfolgenden Testmuster verwendet, bzw. die pH-Werte während den beiden nachfolgenden Testmustern ermittelt, wobei als positive Kontrollen 0,3 mol/L 10%-Saccharose-Spüllösung oder zuckerhaltige Bonbons verwendet worden sind.

| Testmuster 1 Fig. 1 | min |
|---|---|
| - Paraffin-Kauen | 3 |
| - Ruheperiode | 4 |
| - Kontrollperiode | 15 |
| - 15 ml 0,3 mol/L Saccharose-Spülung | 2 |
| - Kontrollperiode | 15 |
| - Lutschen eines 628/2 Bonbons | individuell |
| - Kontrollperiode | 30 |
| - Wasserspülung | 2 |
| - Paraffin-Kauen | 3 |
| - Ruheperiode | 4 |

| Testmuster 2 Fig. 2 | |
|---|---|
| - Paraffin-Kauen | 3 |
| - Ruheperiode | 4 |
| - Kontrollperiode | 15 |
| - 15 ml 0,3 mol/L Saccharose-Spülung | 2 |
| - Kontrollperiode | 15 |
| - Lutschen eines 628/2 Bonbons | individuell |
| - Kontrollperiode | 15 |
| - Wasserspülung | 2 |
| - Paraffin-Kauen | 3 |
| - Ruheperiode | 4 |
| - 15 ml 0,3 mol/L Saccharose-Spülung | 2 |
| - Kontrollperiode | 15 |
| - Kauen eines Candida-Kaugummis | gleich wie Bonbon |
| - Kontrollperiode | 15 |
| - Wasserspülung | 2 |
| - Paraffin-Kauen | 3 |
| - Ruheperiode | 4 |

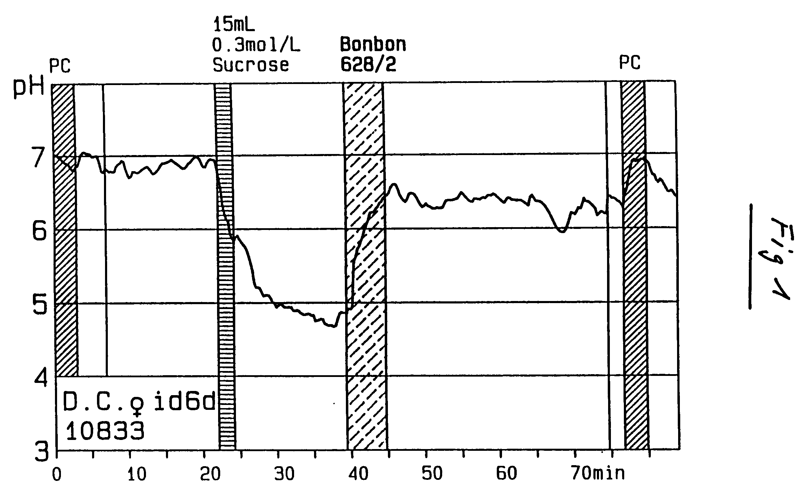

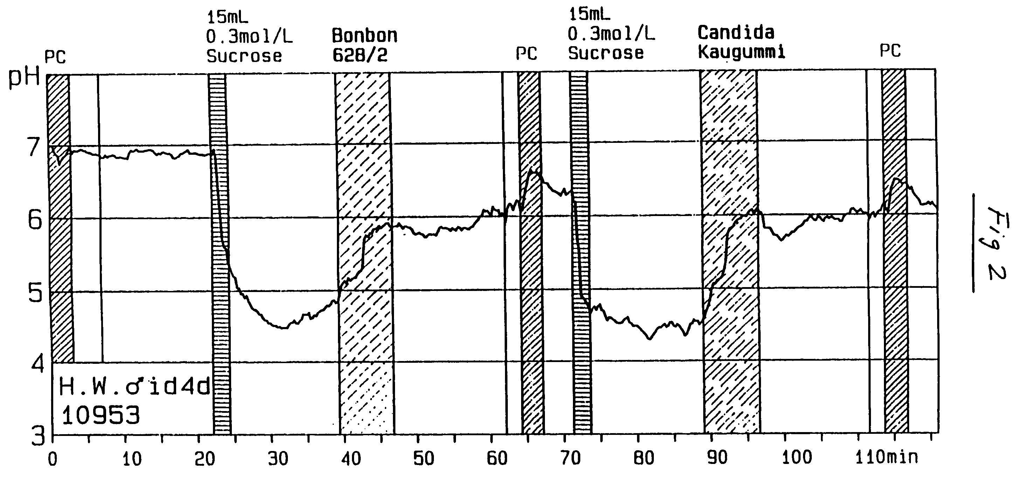

Testmuster 1 wurde durchgeführt zum Nachweis der plaqueneutralisierenden Wirkung und Testmuster 2 zum Vergleich der plaqueneutralisierenden Wirkung eines erfindungsgemässen Bonbons mit derjenigen eines neutralisierenden Kaugummis. Dabei ist das Testmuster 1 in Fig. 1 grafisch dargestellt, währenddem Testmuster 2 grafisch in Fig. 2 dargestellt ist. Dabei ist zu erwähnen, dass selbstverständlich für jeden der 8 Probanden ein entsprechendes Diagramm erstellt worden ist, jedoch stellvertretend immer nur eines in den Fig. 1 und 2 dargestellt ist.

Die Untersuchungsergebnisse gemäss den Fig. 1 und 2 zeigen, dass die dem erfindungsgemässen Bonbon beigefügten puffernden Lebensmittelzusätze in der Lage sind, eine durch Kohlenhydratvergärung im Anschluss an eine Saccharose-Spülung angesäuerte Plaque schnell zu neutralisieren, und damit die schädliche Wirkung der Saccharose zu begrenzen. Fig. 2 zeigt zusätzlich einen Vergleich der plaqueneutralisierenden Wirkung des getesteten Bonbons mit derjenigen eines bereits aus dem Stand der Technik bekannten Kaugummis der Marke Candida. Obwohl Kaugummi durch die mechanische Stimulation (Kaubewegung) eine höhere Speichelfliessrate hervorrufen kann, ist das Bonbon in der Lage durch die ihm zusetzten Puffersubstanzen die gleiche Wirkung zu erzielen.

Selbstverständlich handelt es sich bei der oben angeführten Rezeptur zur Herstellung eines erfindungsgemässen Lutschbonbons bzw. Neutralisationsbonbons nur um ein Beispiel um die Erfindung näher zu erläutern, und um anhand einer erfindungsgemässen Formulierung Tests durchführen zu können. Selbstverständlich kann die Formulierung im Sinne der in den Ansprüchen angeführten Massnahmen abgeändert, variiert oder ergänzt werden. Grundsätzlich ist es überraschend, dass mittels eines Lutschbonbons, beinhaltend eine neutralisierende bzw. reinigende Wirkung praktisch derselbe Effekt erzielt werden kann, wie dies mit einem obgenannten Kaugummi möglich ist. Da die Akzeptanz der Verwendung eines Lutschbonbons wesentlich höher ist als bei einem Kaugummi wird dadurch eine Lösung angeboten, um zumindest in speziellen Situationen bzw. beim Vorliegen spezieller Umstände nach dem Verzehr von Genuss- oder Nahrungsmitteln in der Mundhöhle gebildete Säuren zu neutralisieren. Selbstverständlich wird mittels einem erfindungsgemässen Lutschbonbon nicht das schlussendliche Reinigen der Zähne mittels einer Zahnbürste ersetzt.

## Patentansprüche

1. Lutschbonbon für Zahnbelagsneutralisation mittels einem säureneutralisierenden Wirkstoff, dadurch gekennzeichnet, dass es mindestens einen der nachfolgenden Wirkstoffe enthält:
ein Amoniumsalz, Alkalimetallsalz, Erdalkalimetallsalz, wie insbesondere ein Phosphat, Carbonat, Hydrogencarbonat und/oder Hydrogenphosphat.

2. Lutschbonbon für Zahnbelagsneutralisation mittels einem säureneutralisierenden Wirkstoff, dadurch gekennzeichnet, dass, dass als Wirkstoff Harnstoff vorliegt.

3. Lutschbonbon nach einem der Ansprüche 1 oder 2, dadurch gekennzeichnet, dass es mindestens einen Zuckeraustausch- bzw. einen Zuckerersatzstoff und/oder einen Süssstoff enthält.

4. Lutschbonbon nach einem der Ansprüche 1 bis 3, dadurch gekennzeichnet, dass es Xylit enthält.

5. Lutschbonbon nach einem der Ansprüche 1 bis 4, dadurch gekennzeichnet, dass mindestens eine Trägersubstanz sowie ein oder mehrere geschmacksbildende Aromastoffe enthalten sind.

6. Lutschbonbon nach einem der Ansprüche 1 bis 5, dadurch gekennzeichnet, dass der Anteil an säureneutralisierendem(en) Wirkstoff(en) mindestens 0,1Gew%, vorzugsweise insgesamt zwischen 0,2Gew% und 15Gew%, bezogen auf das Gesamtgewicht des Bonbons, beträgt.

7. Lutschbonbon nach einem der Ansprüche 1 bis 6, dadurch gekennzeichnet, dass der Anteil an säureneutralisierendem(en) Wirkstoff(en) zwischen 0,5Gew% bis 3Gew%, vorzugsweise 1Gew% bis 2,5Gew%, bezogen auf das Gesamtgewicht des Bonbons, beträgt.

8. Lutschbonbon nach einem der Ansprüche 1 bis 7, dadurch gekennzeichnet, dass als Wirkstoff mindestens Di-Amoniumhydrogenphosphat und/oder Natriumhydrogencarbonat vorliegt.

9. Verwendung eines säureneutralisierenden Wirkstoffes zum Herstellen eines Lutschbonbons für Zahnbelags-Neutralisation nach einem der Ansprüche 1 bis 8, dadurch gekennzeichnet, dass üblicherweise für die Herstellung eines Lutschbonbons verwendete Trägersubstanzen, Wasser sowie Zuckerersatzstoffe gemischt bzw. dispergiert und auf 80°C erwärmt werden, anschliessend bei über 160°C gekocht werden, worauf die gekochte Masse mittels eines zusätzlich vorgesehenen Kühlers auf unter ca. 135°C gekühlt wird, damit beim anschliessenden Zudosieren des oder der säureneutralisierenden Wirkstoffe, wie beispielsweise Di-Amoniumhydrogenphosphat oder Natriumhydrogencarbonat, und Xylit diese durch eine zu hohe Massetemperatur nicht zersetzt werden, worauf die aromatisierte Masse beispielsweise auf ein Stahlband ausgetragen und abgekühlt wird, um schlussendlich in Bonbons geprägt zu werden.

10. Vorrichtung zum Herstellen eines Lutschbonbons nach einem der Ansprüche 1 bis 8, gekennzeichnet durch eine üblicherweise für die Herstellung eines Lutschbonbons verwendete Anlage, welche zusätzlich anschliessend an ein Kochgefäss, in welchem die Trägersubstanzen, Wasser, Zuckerersatzstoffe sowie andere Zutaten bei ca. 160°C gekocht und gemischt werden, ein Zusatzkühler vorgesehen ist, in welchem Zusatzkühler die gekochte Masse abgekühlt wird und anschliessend einem Vakuumraum zugeführt wird, in welchem der Masse Feuchtigkeit entzogen wird, worauf in einem sogenannten Inline-Mischer die Masse mit Aromen und den säureneutralisierenden Wirkstoffen beaufschlagt wird.

## Claims

1. Sucking sweet to neutralize plaque with an acid-neutralising active substance, characterised in that it contains at least one of the following active substances:
an ammonium salt, alkaline metal salt, alkaline-earth metal salt such as in particular a phosphate, carbonate, hydrogencarbonate and/or hydrogenphosphate.

2. Sucking sweet to neutralize plaque by means of an acid-neutralizing active substance, characterised in that urea is present as the active substance.

3. Sucking sweet according to any of claims 1 or 2, characterised in that it contains at least one sugar replacement or sugar substitute and/or a sweetener.

4. Sucking sweet according to any of claims 1 to 3, characterised in that it contains xylite.

5. Sucking sweet according to any of claims 1 to 4, characterised in that it contains at least one carrier substance and one or more flavourings.

6. Sucking sweet according to any of claims 1 to 5, characterised in that the proportion of acid-neutralising active substance(s) is at least 0.1 w.%, preferably in total between 0.2 w.% and 15 w.% in relation to the total weight of the sweet.

7. Sucking sweet according to any of claims 1 to 6, characterised in that the proportion of acid-neutralising substance(s) is between 0.5 w.% to 3 w.%, preferably 1 w.% to 2.5 w.% in relation to the total weight of the sweet.

8. Sucking sweet according to any of claims 1 to 7, characterised in that diammonium hydrogenphosphate and/or sodium hydrogencarbonate are present as an active substance.

9. Use of an acid-neutralising active substance to produce a sucking sweet for plaque neutralisation according to any of claims 1 to 8, characterised in that carrier substances normally used for production of a sucking sweet, water and sugar substitutes are mixed or dispersed and heated to 80°C, then boiled at over 160°C whereupon the cooked mass is cooled by means an additional cooler to below 135°C so that on subsequent addition of the acid-neutralising active substances such as for example diammonium hydrogenphosphate or sodium hydrogencarbonate and xylite, these are not destroyed by too high a mass temperature, where the flavoured mass is then output for example onto a steel belt and cooled in order finally to be pressed into sweets.

10. Device for production of a sucking sweet according to any of claims 1 to 8, characterised by a plant normally used for production of a sucking sweet, where also connected to a cooking vessel in which the carrier substances, water, sugar substitutes and other ingredients are boiled and mixed at around 160°C, is fitted an additional cooler in which the cooked mass is cooled and then supplied to a vacuum chamber in which moisture is extracted from the mass, where in a so-called in-line mixer the mass is mixed with flavourings and the acid-neutralising active substances.

## Revendications

1. Bonbon à sucer pour neutraliser la plaque dentaire à l'aide d'une substance active neutralisant les acides, caractérisé en ce qu'il contient l'une au moins des substances actives suivantes :
un sel d'ammonium, un sel d'un métal alcalin, un sel d'un métal alcalino-terreux, en particulier un phosphate, un carbonate, un hydrogénocarbonate et/ou un hydrogénophosphate.

2. Bonbon à sucer pour neutraliser la plaque dentaire à l'aide d'une substance active neutralisant les acides, caractérisé en ce que la substance active est l'urée.

3. Bonbon à sucer selon l'une des revendications 1 et 2, caractérisé en ce qu'il contient au moins un succédané ou un ersatz du sucre et/ou un édulcorant.

4. Bonbon à sucer selon l'une des revendications 1 à 3, caractérisé en ce qu'il contient du xylitol.

5. Bonbon à sucer selon l'une des revendications 1 à 4, caractérisé en ce qu'il contient au moins une substance porteuse, ainsi qu'une ou plusieurs substances aromatiques formant le goût.

6. Bonbon à sucer selon l'une des revendications 1 à 5, caractérisé en ce que la proportion de la ou des substances actives neutralisant les acides représente au moins 0,1 % en poids, de préférence globalement entre 0,2 et 15 % en poids, par rapport au poids total du bonbon.

7. Bonbon à sucer selon l'une des revendications 1 à 6, caractérisé en ce que la proportion de la ou des substances actives neutralisant les acides représente entre 0,5 et 3 % en poids, de préférence 1 à 2,5 % en poids, par rapport au poids total du bonbon.

8. Bonbon à sucer selon l'une des revendications 1 à 7, caractérisé en ce qu'il contient en tant que substance active au moins de l'hydrogénophosphate diammonique et/ou de l'hydrogénocarbonate de sodium.

9. Utilisation d'une substance active neutralisant les acides pour préparer un bonbon à sucer pour la neutralisation de la plaque dentaire selon l'une des revendications 1 à 8, caractérisée en ce que des substances porteuses habituellement utilisées pour la préparation d'un bonbon à sucer, de l'eau, ainsi que des succédanés du sucre sont mélangés ou dispersés et chauffés à 80°C, puis cuits à une température supérieure à 160°C, après quoi la masse culte est refroidie en dessous d'environ 135°C à l'aide d'un réfrigérant supplémentaire, de telle sorte que, lors de l'addition ultérieure de la ou des substances actives neutralisant les acides, telles que par exemple l'hydrogénophosphate diammonique ou l'hydrogénocarbonate de sodium, ainsi que du xylitol, ces derniers ne soient pas décomposés par une température trop élevée de la masse, après quoi la masse aromatisée est déposée par exemple sur une bande d'acier et refroidie pour être finalement moulée à l'emporte-pièce sous la forme de bonbons.

10. Appareil pour fabriquer un bonbon à sucer selon l'une des revendications 1 à 8, caractérisé par une installation habituellement utilisée pour la fabrication d'un bonbon à sucer, dans laquelle un réfrigérant supplémentaire est prévu après une cuve de cuisson dans laquelle les substances porteuses, l'eau, les succédanés du sucre ainsi que d'autres additifs sont cuits à environ 160°C et mélangés, réfrigérant dans lequel la masse culte est refroidie puis transférée dans une chambre sous vide dans laquelle l'humidité de la masse est éliminée, après quoi la masse est admise dans un mélangeur dit en ligne avec des arômes et des substances actives neutralisant les acides.
